(19) Europäisches Patentamt

European Patent Office

Office européen des brevets

(11) EP 1 339 006 A1

(12) EUROPEAN PATENT APPLICATION

(43) Date of publication:
27.08.2003 Bulletin 2003/35

(51) Int Cl.⁷: G06F 19/00

(21) Application number: 02003469.0

(22) Date of filing: 14.02.2002

(84) Designated Contracting States:
AT BE CH CY DE DK ES FI FR GB GR IE IT LI LU MC NL PT SE TR
Designated Extension States:
AL LT LV MK RO SI

(71) Applicant: AgeLab Pharma GmbH
20251 Hamburg (DE)

(72) Inventors:
• Grabe, Niels, Dr.
20255 Hamburg (DE)

• König, Matthias, Dr.
20144 Hamburg (DE)

(74) Representative: Bohmann, Armin K., Dr.
Bohmann & Loosen,
Anwaltssozietät,
Sonnenstrasse 8
80331 München (DE)

(54) **Method for the detection of a functional protein sequence and an apparatus therefor**

(57)   The present invention is related to a method for the detection of an unknown functional protein sequence within a given protein sequence having a number of amino acids comprising the following steps:

a) providing said given protein sequence having a number of amino acids wherein functional sites shall be predicted;
b) providing a number of known functional protein sequences having a number of contiguous amino acids wherein said known functional protein sequences include proven functional sites;
c) for each of said known functional protein sequences, evaluating a resemblance score at one ore more alignment positions between the known functional protein sequences and a portion of said given protein sequence which is to be analysed;
d) if said resemblance scores exceeds a minimum resemblance threshold, selecting the known functional protein sequence(s) and their alignment position(s);
e) assigning each of the selected known functional protein sequence to a segment of said given protein sequence according to their alignment position(s) wherein said segment is defined by said one or more alignment positions;
f) for each of the segments, creating a matrix containing the number of occurrences of each amino acid at a specific position of said given protein sequence aligned with the respective segment,
g) for each of the matrices, evaluating an overall similarity between the known functional protein sequences assigned to the respective segment and the portion of the given protein sequence related to said evaluated segment, and evaluating a conservation value rating the occurrence of conserved amino acids within the evaluated segment.

Fig. 1

EP 1 339 006 A1

**Description**

The present invention concerns a method and a system for the prediction of short functional significant protein sequences. In particular, the invention is related to a method of predicting phosphorylation sites in protein sequences. The invention is based on a case-based, on-the-fly model generation for prediction.

The invention is described by the example of predicting phosphorylation sites in unknown protein sequences but is applicable for the prediction of any functional significant protein sequence in a longer protein sequence to be analysed.

Phosphorylation is one of the most important mechanisms in the regulation of cellular processes by the post-translational modification of enzymes, receptors and other proteins. Phosphorylation is a chemical process in which a phosphate group is added to an organic molecule associated with respiration and photosynthesis in living cells. The prediction of unknown functional sites, especially of unknown phosphorylation sites, is difficult as functional sites, especially phosphorylation sites, are usually short in length (not longer than about ten amino acid residues). Due to the short length common sequence alignment tools, e.g. the BLAST algorithm as described in Altschul S.F. and Lipman D.J. (1990), Basic Local Alignment Search Tool, J.Mol. Biol. 215, 404-410, or FASTA as described in Pearson W. R. and Lipman D. J. (1988), Improved Tools for Biological Sequence Analysis, Proc. Natl. Acad. Sci 85, 2444-2448, yield no useful results because of a large number of irrelevant predictions.

For the prediction of functional patterns in protein sequences, the motif approach emerged in methods like PROSITE as described in Hofmann K., Bucher P., Falquet L., and Bairoch A. (1999), *The PROSITE database, its status in 1999,* Nucleic Acids Res. 27, 215-219, BLOCKS as described in Henikoff S., Henikoff J.G. and Pietrokovski S. (1999), Blocks+: A non-redundant database of protein alignment blocks derived from multiple compilations, Bioinformatics 15, 471-479, or PRINTS as described in Attwood T.K., Flower D.R., Lewis A.P., Mabey J.E., Morgan S.R., Scordis P., Selley J., and Wright, W. (1999), *PRINTS prepares for the new millennium*, Nucleic Acids Res. 27, 220-225, which use local sequence patterns (or motifs) to help identifying function or activity of proteins. Local sequence information means several contiguous amino acid residues. PROSITE is representative of the general motif approach.

PROSITE is a method to determine the function of not characterized proteins translated from genomic or cDNA sequences. It comprises a database of biologically significant sites and patterns formulated so that it can be rapidly and reliably identified by appropriate computational tools to which known protein family (if any) the new detected sequence is assigned. A pattern describes a group of amino acids that constitutes an usually short but characteristic motif within a protein sequence. The pattern syntax allows the expression of ambiguities, for example if two different amino acids are allowed at a given position. For example, the pattern

[AC]-x-V-x(4)-{ED}

is interpreted as [Ala or Cys]-any-Val-any-any-any-any-{any but Glu or Asp}. In addition to patterns a variant of PROSITE uses profiles as described more detailed in the PROSITE User Manual.

A profile, i.e a weight matrix, is a table of position-specific amino acid weights and gap costs. These numbers are used to calculate a similarity score between the profile and a sequence to be analysed. A similarity score higher than or equal to a given cut-off value constitutes a motif occurrence. Profiles can be constructed by a large variety of different techniques. The classical method is disclosed in Gribskov M. and Devereux J. (1992), *Sequence analysis primer,* Freeman and Company, and requires a multiple sequence alignment as input. It uses a symbol comparison table to convert distributions of the number of residue occurrences into weight values. Profiles are supposed to be more sensitive than patterns because they individually assign weights to single residues, while pattern approaches can only be applied to detect matching or non-matching with a single residue.

Instead of profiles or weight matrices also hidden Markov models (HMMs) or artificial neural networks (NN) can be used. PFAM (Protein Families Database of Alignments) is a collection of protein motifs and families, see also Bateman A., Birney E., Durbin R., Eddy S.R., Howe K.L., Sonnhammer E.L. (2000), The *Pfam Protein Families Database,* Nucleic Acids Research 28, 263-266. It is possible to convert a PFAM HMM into a profile, both resulting in nearly the same similarity scores when applied to the same sequence. Although a few minor technical differences subsist for practical applications, PFAM HMMs and PROSITE profiles are substantially mutually convertible. However, this cannot be extended to other applications involving HMMs. Many biological motifs are described both by a PROSITE profile and a PFAM HMM. The results generated by these two models and methods, respectively, are usually different. This is probably caused by the different biological considerations which are applied for the multiple alignment on which both methods substantially rely. In a similar way weight matrices (short 'matrices') can be applied to certain architectures of neural networks where each weight of a synapse of the neural network corresponds to a specific value of a weight matrix. Introducing an additional layer to a neural network then corresponds to a matrix where each layer represents a combination of several amino acids.

An implementation of neural networks for the prediction of phosphorylation sites is realised by NETPHOS that is described in Blom N., Gammeltoft S. and Brunak S. (1999), Sequence and Structure-based Prediction of Eukaryotic Protein Phosphorylation Sites, J. Mol. Biol. 294, 1351-1361. While approaches like PROSITE use a whole set of patterns or matrices NETPHOS establishes three separate neural networks, each for one of three phosphorylation acceptor residues, namely tyrosine (Y), serine (S) and threonine (T). In the document Blom N., Gammeltoft S. and Brunak S. (1999), Sequence and Structure-based Prediction of Eukaryotic Protein Phosphorylation Sites, J. Mol. Biol. 294, 1351-1361, the construction of individual neural networks for individual groups of known phosphorylation sites is mentioned.

Two of the above described approaches are commonly applied NETPHOS and PROSITE patterns.

The NETPHOS approach advantageously provides automatic learning. The neural network incorporates all known functional protein sequences in one model, e. g. the phosphorylation sites of one acceptor residue (T, S or Y). There is no need of manually maintaining and using several sets of models.

On the other hand, the pattern/matrix approach leads to more accurate and individual models for the prediction of phosphorylation sites.

Both approaches, the NETPHOS implementation and the pattern/matrix approach, have the drawback of relying on predefined models including known phosphorylation sites which had been compiled previously by more or less unknown conditions or circumstances. The models are manually predefined which may result in predictions with different accuracy. Therefore, a general uncertainty and unavoidable bias and error is to expect in the prediction models. The bias and error necessarily lead to a higher uncertainty in prediction which will automatically result in a reduced sensitivity and specificity of the predictions.

The problem underlying the present invention is therefore to provide a method for the detection of unknown functional protein sequences within a given protein sequence which overcomes the drawbacks of the methods of the prior art.

In a first aspect the problem underlying the present invention is solved by a method for the detection of an unknown functional protein sequence within a given protein sequence having a number of amino acids comprising the following steps:

a) providing said given protein sequence having a number of amino acids wherein functional sites shall be predicted;

b) providing a number of known functional protein sequences having a number of contiguous amino acids wherein said known functional protein sequences include proven functional sites;

c) for each of said known functional protein sequences, evaluating a resemblance score at one ore more alignment positions between the known functional protein sequences and a portion of said given protein sequence which is to be analysed;

d) if said resemblance scores exceeds a minimum resemblance threshold, selecting the known functional protein sequence(s) and their alignment position(s);

e) assigning each of the selected known functional protein sequence to a segment of said given protein sequence according to their alignment position(s) wherein said segment is defined by said one or more alignment positions;

f) for each of the segments, creating a matrix containing the number of occurrences of each amino acid at a specific position of said given protein sequence aligned with the respective segment,

g) for each of the matrices, evaluating an overall similarity between the known functional protein sequences assigned to the respective segment and the portion of the given protein sequence related to said evaluated segment, and evaluating a conservation value rating the occurrence of conserved amino acids within the evaluated segment.

In an embodiment of the inventive method the alignment positions of one of said known functional protein sequences are selected if the resemblance score of one of said known functional protein sequence has a maximum resemblance score relating to said maximum resemblance scores at all alignment positions.

In a preferred embodiment of the inventive method step c) of evaluating said resemblance scores at one ore more alignment positions includes the evaluation of said resemblance scores at all alignment positions.

In another embodiment of the inventive method it is envisaged that according to step e) said selected known functional protein sequences are assigned into the same segment if their selected alignment positions are equal.

In a further embodiment of the inventive method it is envisaged that according to step e) said selected known functional protein sequences are assigned into the same segment if their selected alignment positions are within a predetermined segmentation range.

In still a further embodiment of the inventive method it is envisaged that successive known functional protein sequences are assigned to the same segment if the distance of said successive known functional protein sequences is less than half of the matrix length.

In a preferred embodiment of the inventive method the evaluation of the overall similarity of step g) is performed only if the number of known functional protein sequences assigned to one segment exceeds a predetermined threshold number of known functional protein sequences, otherwise the segment is discarded.

In a more preferred embodiment of the inventive method it is envisaged that the predetermined threshold number of known functional protein sequences is at least 3.

In a further embodiment of the inventive method it is envisaged that the evaluation of the overall similarity of step g) further includes the step of

for each of said matrices, evaluating said overall similarity using the formula

$$\textbf{overallsim} := overall\_similarity(ups, \text{m}) := \prod_{l=1}^{s} \left( \frac{p_{lAl}}{p_{lO}} \right).$$

wherein ups is the specific portion of the given protein sequence aligned with the respective segment,
wherein m is the matrix,
wherein s is the length of the specific portion of the given protein sequence,
wherein p10 is the maximum number of occurrences of a specific amino acid, and
wherein $p_{lAl}$ is the number of occurrences of the amino acid at the respective position of the specific portion of the given protein sequence.

In another embodiment of the inventive method the evaluating of said conservation value *conservation* rating the occurrence of conserved amino acids within the evaluated segment is performed using the formula

$$conservation := 1/s * \sum_{l} \left( \log_{base}(20) + \sum_{lA} p_{lA} * \log_{base}(p_{lA}) \right)$$

wherein s is the total number of possible amino acids for each of the positions,
wherein base is the basis of the logarithm, preferably 2 or 10,
wherein $p_{lA}$ denotes the evaluated probability of the occurrences of the specific amino acid in the respective column l of said matrix.

In a further embodiment of the inventive method step e) of assigning each of the selected known functional protein sequence to a segment of said given protein sequence according to their alignment position(s) is performed if said overall similarity of the segment including the assigned known functional protein sequence exceeds the overall similarity of the segment without said known functional protein sequence, and/or if the conservation value of said segment including the assigned known functional protein sequence exceeds the conservation value of said segment without said assigned known functional protein sequence.

In another aspect the problem underlying the present invention is solved by a method for the verification of an unknown functional protein sequence comprising the steps of the inventive method for the detection of an unknown functional protein sequence within a given protein sequence having a number of amino acids, resulting in an annotated unknown functional protein sequence whereby the annotated unknown functional protein sequence is a functional protein sequence having a minimum overall similarity and/or a minimum conservation value,
further comprising the step of

experimentally verifying the annotated unknown functional protein sequence.

In still another aspect the problem underlying the present invention is solved by the use of any of the inventive methods for predicting phosphorylation sites in a given protein sequence, wherein the known functional protein sequences contain proven phosphorylation sites.

In a further aspect the problem underlying the present invention is solved by an apparatus for the detection of an unknown functional protein sequence within a given protein sequence,
wherein said given protein sequence includes a number of amino acids wherein functional sites shall be predicted,
wherein a number of known functional protein sequences having proven functional sites are provided which include a number of contiguous amino acids, comprising:

- means for evaluating a resemblance score between the known functional protein sequences and a portion to be analysed of said given protein sequence at one ore more alignment positions for each of said known functional protein sequences;

- selecting means to select the known functional protein sequence and their alignment positions, if said resemblance scores exceed a minimum resemblance threshold,
- assigning means to assign each of the selected known functional protein sequences to a segment according to their alignment position wherein said segment is defined by said one or more alignment positions,
- overall similarity evaluating means to evaluate the overall similarity between the known functional protein sequences assigned to the respective segment and the portion of the given protein sequence related to said evaluated segment for each of the segments.

In a preferred embodiment the inventive apparatus further comprises:

- overall conservation evaluating means to evaluate the conservation value for each of the segments wherein the conservation value rates the occurrence of conserved amino acids within the evaluated segment.

The present invention provides a method for the detection of a - so far - unknown functional protein sequence within a given protein sequence having a number of amino acid residues. It is provided that the given protein comprises an amino acid sequence (also referred to herein as protein sequence), wherein functional sites such as phosphorylation sites shall be predicted. The term unknown functional (protein) sequence as used herein shall mean that a given sequence comprises a site, i. e. one or more amino acid residues, which is functional, preferably in vivo, whereby the fact that it is functional is not known prior to the application of the present method. In contrast to this a known functional (protein) sequence is a sequence comprising a site, i. e. one or more amino acid residues, which is functional, preferably in vivo, whereby the fact that it is functional is known prior to the application of the present method. As used herein, the term functional means post-translational processing, protein sorting, protein function etc. Therefore functional sites can be cleavage sites (protease), phosphorylation and other modification sites (protein kinase etc. ), ATP binding sites, signal sequences (signal recognition particle), localization signals, DNA binding sites (DNA), Ligand binding sites (ligands), catalytic sites etc.

Further, a number of known functional protein sequences having a number of contiguous amino acids is provided wherein said known functional protein sequences include proven or confirmed functional sites. For each of said functional protein sequences a resemblance score between the known functional protein sequence and the portion of said given protein sequence to be analysed at one or more alignment positions of the given protein sequence to be analysed is evaluated. The known functional protein sequences and their alignment positions are selected if said resemblance scores exceed a minimum resemblance threshold.

According to the alignment position each of the respective known functional protein sequences is designed into a segment wherein the segment is defined by said one or more alignment positions. For each of the segments the overall similarity between the known functional protein sequences assigned to the respective segment and the portion of the given protein sequence related to said evaluated segment is evaluated.

The method of the present invention allows to automatically construct models of known functional protein sequences which are highly similar to the functional sites to be detected. According to the present invention the prediction is done by the model construction, while in the prior art models are built in advance and applied later.

The method of the present invention overcomes the inaccuracy evolving from the prediction appearing from the short length of the known functional protein sequences and the functional protein sequences to be found. The case-based model construction allows a high degree and sensitivity of evaluation.

In view of their inventive method it is no longer necessary to maintain numerous predefined different models of binding sites because according to the method of the present invention case-based models can be automatically constructed.

The case-based model construction is only controlled by a few specific parameters that allow to obtain predictions with constant sensitivity and specificity. The specific parameters are applied in the same manner for all models which results in predictions of constant accuracy. In contrast to this, all models of the prior art are constructed in different manners at different times by different users. Accordingly, different independent users of such a model are not able to find out the parameters on which the former prediction method is based and are therefore not able to get or rely on the same parameters for applying the model.

Also, the invention solves several of the currently existing problems in the art:

- overcoming the limitations caused by the shortness of phosphorylation sites,
- maintaining many predefined different models of binding sites,
- obtaining highly sensitive predictions, and
- obtaining predictions with a constant sensitivity and specificity.

Further embodiments of the present invention may be taken from the dependent claims.

Preferably, the alignment positions of one of said known functional protein sequences are selected if the resemblance score of one of said known functional proteins sequence has a maximum resemblance score wherein the maximum resemblance score is determined by the maximum value of all resemblance scores at all alignment positions of one known functional sequence. So a smaller number of aligned known functional protein sequences is selected with the given protein sequence for further processing. This increases the accuracy of prediction and the processing speed but may also have the effect that less unknown functional sites are found, resulting in more distinct prediction results.

According to another preferred embodiment of the present invention the evaluation of said resemblance scores at one or more alignment positions includes the evaluation of said resemblance scores for all alignment positions. This will be preferred if no pre-selection of specific alignment positions to be analysed is made or can reasonably be made.

In a preferred embodiment said selected known functional protein sequences will be assigned to the same segments if the selected alignment positions assigned to known functional protein sequences are equal. This allows to further increase the accuracy of the prediction methods. It also speeds up processing because functional protein sequences which are aligned at a single and at a specific position of said given protein sequence, can be discarded and, thus, are no longer involved in further processing.

It may also be possible to assign said selected known functional protein sequences to the same segment if their selected alignment positions are within a predetermined segmentation range. This allows to assign more selected known functional protein sequences to one segment, thereby providing a segment which has a sufficient support, i.e. more than a specific number of known functional protein sequences are assigned to one segment.

Preferably the evaluation of the overall similarity between the known functional protein sequences assigned to the respective segment and the portion of the given protein sequence **ups** related to said evaluated segment is performed by creating a matrix. For each of the segments the respective matrix is created containing the number of occurrences of each amino acid at a specific position of said given protein sequence aligned with the respective segment.

For each of said matrices m the overall similarity is evaluated according to the following formula:

$$\textbf{overallsim} := overall\_similarity(ups, \mathrm{m}) := \prod_{l=1}^{s} \left( \frac{p_{lAl}}{p_{lO}} \right).,$$

wherein s is the length of the portion of the protein sequence to be analysed,

wherein $p_{lAl}$ is the number of occurrences of the specific amino acid at a specific position **l** of said given protein sequence aligned with the respective segment,

wherein $p_{lO}$ is the number of occurrences of the most frequent amino acid at the position s of the given protein sequence to be analysed.

This allows to evaluate the overall similarity related to each of the segments in a fast and easy manner. The overall similarity is calculated using the matrix m in which the number of occurrences of each amino acid at each of the positions s of the given protein sequence lps to be analysed is included. The overall similarity is evaluated by the product of the number of occurrences of the respective amino acids of the given protein sequence lps at each position to be analysed divided by the product of the maximum number of occurrences of any of the amino acids at every position of the given protein sequence to be analysed. Thereby it is possible to determine an overall similarity taking into account all known functional protein sequences which are similar to the portion of the given protein sequence lps.

It is preferred to determine a conservation value for each of said segments rating the occurrence of the conserved amino acids within the evaluated segment using the formula:

$$C_l = \log_2 (20) + \sum_{lA} p_{lA} * \log_2(p_{lA})$$

$$conservation := 1/s* \sum_{l} C_l$$

Wherein $\mathbf{p_{lA}}$ denotes the evaluated probability of the occurrences of the specific amino acid A in the the respective column l of said matrix m, and

wherein $\mathbf{C_l}$ denotes the conservation of column **l** in the matrix ($\mathbf{p_{lA}}$) with the length s of the unknown protein sequence to be analysed. The sum covers all amino acids in the respective column **l** of the matrix. The calculated column conservation $C_l$ describes the certainty of occurrence of only one amino acid in column **l** of the matrix. From the single $C_l$ the matrix conservation *conservation* is derived as the average over all columns **l** from 1 to **s**. The matrix conservation *conservation* represents the confidence of the matrix provided for the prediction, i.e. the likelihood or reliability that the matrix represents a functional site.

The method of the present invention can be advantageously applied for predicting phosphorylation sites in a given protein sequence wherein the known functional protein sequences containing proven phosphorylation sites are used. This is especially preferred as the phosphorylation sites are small and thereby not predictable with convenient accuracy using methods according to the prior art as described before.

According to another aspect of the present invention an apparatus for detection of an unknown functional protein sequence within a given protein sequence is provided. The apparatus comprises means for evaluating a resemblance to evaluate a resemblance score between the known functional protein sequence and the portion of said given protein sequence to be analysed at one ore more alignment positions for each of said functional protein sequences. The apparatus further comprises selecting means to select the known functional protein sequence and their alignment positions, if said resemblance scores exceed a minimum resemblance threshold. To assign each of the selected known functional protein sequences to a segment according to their alignment position an assigning means is provided wherein said segment is defined by said one or more alignment positions. By providing an overall similarity evaluation means the overall similarity between the known functional protein sequences assigned to the respective segment and the portion of the given protein sequence related to said evaluated segment can be evaluated for each of the segments.

The apparatus of the present invention allows the performing of the inventive method as disclosed herein and can be realized by a standard computer running a computer program code establishing the elements of the apparatus.

It is to be understood that the annotation made to a part or segment of a given protein sequence to be a functional site as obtained by the inventive method may be further verified. Such verification can be done by various experimental procedure such as further in silico, in vivo, in vitro and/or in situ testing. Once this annotation is available the ones skilled in the art may use it for, e. g., target validation, drug design, diagnostic purposes, therapeutic purposes, metabolic design, gene therapy, and basic research.

As used herein the term "to evaluate a value" shall preferably mean to determine a value.

The method and apparatus of the present invention are now explained and illustrated in more detail especially by the following figures and examples relating to the prediction of phosphorylation sites. It is to be acknowledged that from the figures and the examples further features, embodiments and advantages of the present invention may be taken, whereby.

Fig. 1    illustrates the concept of lps, pps, ups, apps, block and segment, and

Fig. 2    depicts the flow starting with *pps* and resulting in matrices.

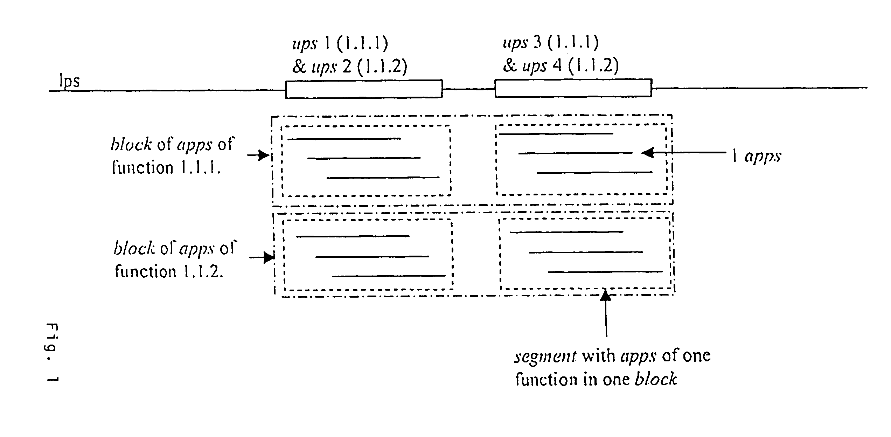

Fig. 1 illustrates the concept of lps, pps, ups, apps, block and segment. Proven phosphorylation sites (pps) are first aligned to the longer protein sequence (lps) yielding aligned proven phosphorylation sites (apps) corresponding to the unknown phosphorylation sites (ups 1 - 4) where ups 1 - 2 and ups 3 - 4 are at exactly the same position in lps in this example. Then the apps are split by their respective function into blocks and then split by position into segments. Such function may be or may be related to, preferably in the hierarchical system as preferably used in the method according to the present invention, cleavage sites (protease), phosphorylation and other modification sites (protein kinase etc.), ATP binding sites, signal sequences (signal recognition particle), localization signals, DNA binding sites (DNA), Ligand binding sites (ligands), catalytic sites and the like. This results in apps with the same function at the same position,

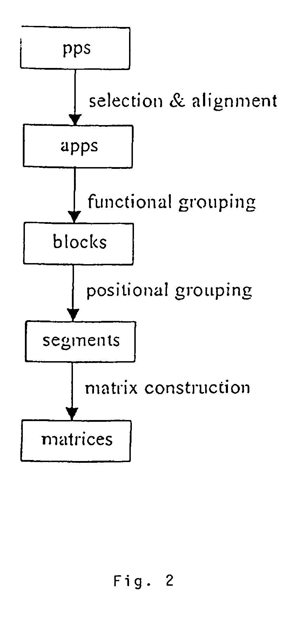

Fig. 2 depicts the flow starting with *pps* and resulting in matrices. The *pps* are aligned to *lps* resulting in *apps*. *Apps* are grouped by their function and assigned to segments according to their position. Matrices are built from the resulting segments.

The method and the apparatus of the present invention uses a database **D** (e.g. the Phosphobase [Kreegipuu A., Blom N. and Brunak S. (1999), *PhosphoBase, a database of phosphorylation sites: release 2.0.,* Nucleic Acids Res 27(1), 237-239]) of biologically proven phosphorylation sites (*pps*) to predict unknown phosphorylation sites *(ups)* in a given longer protein sequence (*lps*) **u.** The method includes the steps listed below. The principle is applicable for other kinds of short functional sequence elements to be detected in longer protein sequences to be analysed.

1. Preprocessing: *pps* are extracted from **D** and grouped by the three possible acceptor residues (tyrosine, serine and threonine) and subcategorised by kinase classes (e.g. every PKC phosphorylation site is assigned to one category that is a subclass of serine).

2. Alignment: All *pps* are aligned to the protein sequence *lps* to be analysed yielding a set of aligned *pps* (*apps*).

3. Segmentation: The aligned known binding sites (*apps*) taken from the database **D** are grouped in segments **G** according to their function (i.e. acceptor residue and kinase class) and position relative in *lps*. Each segment of **G,** containing *apps* of one function and a similar position, ideally predict one single unknown phosphorylation site *ups* in the longer protein sequence *lps* under evaluation.

4. Model construction: All *apps* contained in one segment are hierarchically aligned leading to a set of matrices in each segment. Each matrix comprises a subset of the *apps* in the segment under study. All *apps* of those subsets are highly similar to each other, the according matrix is highly similar to the *ups* (i.e. the consensus, described by the matrix, resembles the *ups*) and the matrix is highly conserved (the variance in each column of the matrix is low).

In the following, the above mentioned steps are described in more detail.

The alignment step is performed aligning all known phosphorylation sites pps with the given protein sequences lps at each possible position s. The known binding sites pps are so aligned to the unknown protein sequence u*ps*. An evaluation of the resemblance is performed by introducing a score which constantly rises when successive amino acids of the analysed sequence are matching with the phosphorylation site. This resemblance score is then assigned to each of the amino acids of the analysed sequence. The evaluation ensures that a contiguous sequence of amino acids of length **l** is higher than two runs of length **a** and **b** with **l** = **a** + **b**. The resemblance is the sum of the scores of every position of the amino acids. With **n** as the length of the known phosphorylation site and the unknown *lbs* **ups** one gets:

$$resemblance\ (u, s) := \Sigma_{i=1..n}\ \eta *\ SMA$$

where SMA is the number of successive matching amino acids

## Example ($\eta$=2):

```
Examined Sequence ups:              L K L A S P E L E L K
Known phosphorylation site pps:     E K L A S K E L E V D
SMA                                 0 4 4 4 4 0 3 3 3 0 0
SMA * η                             0 8 8 8 8 0 6 6 6 0 0
```
$$\underbrace{\qquad}_{SMA=4} \quad \underbrace{\qquad}_{SMA=3}$$

As the first examined amino acid is non-matching a SMA value of "0" is assigned to the first position. For the first matching portion of the examined sequence the SMA equals 4 as four successive matching amino acids "KLAS" are detected before the next examined amino acid is non-matching. Thus to each of the matching amino acid residue a SMA number of 4 is assigned. The next occurring sequence of matching amino acids has a length of 3 so a SMA value of 3 is assigned to each of the matching amino acids.

The total resemblance score according to the above given formula is the sum of all SMA values weighted by the $\eta$ value of "2" which is user defined and can have other values.

For the upper example the total resemblance score is 4*8+3*6=32+18=40.

This step results in a set **P** of aligned phosphorylation sites (*apps*) wherein the known phosphorylation sites are associated with a resemblance score. To reduce the number of elements in set P only elements whose resemblance score is exceeding a given threshold *resemblance* may be considered and added to set P. This allows to control the number of elements in P and to gain processing speed for the following steps. The threshold resemblance is user defined and set to control the number of elements of set P for the following steps.

To construct matrices the set **P** has to be sorted by function and position. The function is classified regarding the two levels shown in Table 1.

Table 1:

| Classification of phosphorylation sites | | | |
|---|---|---|---|
| Level | Name | Criterion | Example |
| 1 | Class | Acceptor residues | S-14 |
| 2 | Subclass | Kinase | PKC |

The elements of set P are assigned to different blocks **B** according to the respective levels.

The alignment **p** ∈ **P** of the unknown functional protein sequence **u** and a binding site is described by the pair <site_id, site_class>.

*Example:* The set **P** = {<T0001, 2.1>, <T0002, 1.1>, <T0003, 2.2>, <T0004, 2.1>, <T0005, 1.2>} yields to the blocks **B₁, B₂** with **B₁** = {<T0002, 1.1>, <T0005, 1.2>} and **B₂** = {<T0001, 2.1>, <T0004, 2.1>, <T0003, 2.2>}.

Each block **B₁, B₂** is split into segments **G** wherein the position of each **p** ∈ **P** is used to assign the element pairs of each block to the respective segments according to the position in the unknown functional protein sequence **u** to be analysed. If the respective positions **p₁, p₂** ∈ **B** of one of the blocks are distanced to each other for more than half the desired matrix width **p₁** and **p₂** are put into separate segments **G** otherwise they are assigend to the same segment G. Therefore, each segment **G** comprises the element pairs including the positions of possible binding sites that are bound to similarly structured domains and found at similar positions.

In a next step matrices have to be constructed. The construction of the matrices is done using a gap free heuristic alignment. Gap free heuristic alignment means that no gaps in the matrix construction are considered. A heuristic algorithm is used because an optimal alignment is NP-complete. Gaps cannot be taken into consideration because of the short length of the phosphorylation sites. The heuristic alignment aligns the phosphorylation sites of each of the segments **G** in the order of the resemblance score of each found possible phosphorylation site. Phosphorylation sites at the same position of the given protein sequence are assigend to one matrix while several matrices are constructed if the found phosphorylation sites partially overlap each other or do not overlap.

Adopting the strategy of hierachical clustering, possible phosphorylation sites of **G** are successively merged to a large matrix until the matrix fulfills specific requirements, i.e. the matrix's average *conservation* value should be above **a** given minimum conservation *min_con*, the matrix should have a minimum *overall_similarity* to **u** *min_sim*, and there should be a minimum number of sites *min_sup* in one of the matrices to *support* the sequence.

The *conservation* value is calculated by the average information content given by:

$$C_l = \log_2(20) + \sum_{lA} p_{lA} * \log_2(p_{lA})$$

$$conservation := 1/s * \sum_l C_l$$

$$\underline{\text{Eq. 1}}$$

Here **C_l** denotes the conservation of column **l** in the matrix (**P_{lA}**) with the length s of the unknown protein sequence to be analysed. The sum covers all amino acids in the respective column **l** of the matrix. The calculated column conservation **C_l** describes the certainty of occurrence of only one amino acid in column **l** of the matrix. From the single **C_l** the matrix conservation is derived as the average over all columns **l** from 1 to **s**.

The overall similarity between matrix and ups is determined as follows.

The overall similarity between the matrix and *ups* is calculated as disclosed in the document Berg, O. G. and P. H. von Hippel (1987), *Selection of DNA binding sites by regulatory proteins. Statistical-mechanical theory and application to operators and promoters.*, J. Mol. Biol. 193, 723-750:

The reduction in free binding energy is

$$r(u) := \sum_{l=1}^{s} E_{lA} \text{ with } -E_{lB} * \lambda = \ln\left(\frac{p_{lA}}{p_{lO}}\right) \text{ with } \lambda = 1,$$

$$\underline{\text{Eq. 2}}$$

where **E_{lA}** is the binding energy for a single amino acid. The probability **p_{lA}** is given by the number of the amino acid corresponding the unknown functional protein sequence *ups* to be analysed. **p₁₀** is largest value in column **l**, i.e. the largest number of occurrences of one of the possible amino acids, and reflects the consensus sequence. This leads to the following binding energy for *ups:*

$$energy\ (ups, m) := K_0 * \exp(-r(ups)),\qquad \text{Eq. 3}$$

where $K_0$ is a binding constant and matrix $m = (p_{IA})$. In general $K_0$ is assumed to equal 1 if not known. Using $sim = energy(ups, m)$ leads to the calculation for the overall similarity **sim** of the matrix **m** related to an unknown partial protein sequence *ups* having a length **s** and with $p_{IO}$ as a maximum of a column as follows:

$$\mathbf{sim} := overall\_similarity(ups, m) := \prod_{l=1}^{s}\ (\frac{p_{IAl}}{p_{IO}}).$$

<u>Eq. 4</u>

The matrix is then constructed according to the following flow program. Each found phosphorylation site is assigned to an existing matrix or to a different matrix. Matrices which are poorly supported as determined by a users variable min_sup are discarded after finishing the assignment of all found possible phosphorylation sites.

```
1  M := {generate_matrix(p)}, p is first apps of segment
2      for all other p ∈ G {
3         for all m ∈ M {
4            if (p and m overlap) and (p not already used for m) {
5               m' := m merged with p
6               if (overall_similarity(u,m')>=min_sim) and (conservation(m') >= min_cons) {
7                  m := m' }
8               else {
9                  M := M ∪ generate_matrix(p) }}}}
10 for all m ∈ M
11    if support(m) < min_sup [
12       discard m }}
```

<div align="center">Method of matrix construction</div>

*generate_matrix(p)*

```
1      P := {set of apps in matrix } ∪ p
2      m' := empty matrix
3      for all p' ∈ P {
4            m' := m' extended by p' }
5      m := window with width matrixlen with highest conservation in m'
```

<div align="center">Method of matrix generation</div>

The matrix **m'** emerges from the already generated matrix **m** extended by the element pair at position **p**. The width of each matrix is chosen by a parameter and normally is the same for each matrix although phophorylation sites can differ in length. It is also possible to choose different width for the matrices.

As a minimal conservation is required for each of the matrices a parameter *min_cons* is introduced which assures that a minimal amount of information is available to predict the existence of a binding site. Accordingly, the parameter *min_sim* denotes the minimal required similarity and the parameter *min_sup* denotes the minimal required support of each of the matrices.

A new matrix is generated if the overall similarity or conservation value of an existing matrix **m** would decrease by merging **p** to **m.** Hence, overall similarity and conservation values are evaluated each time a new element pair is merged. The new matrix (line 9) is added to M. At last the algorithm for generating the extended matrix: generate_matrix (p) is described.

The variable input parameters of the total method are

- $\eta$**:** used for adjusting the resemblance score in "alignment"
- **matrixlen:** desired width of matrix, used in "matrix construction"

The following parameters are used as minimum thresholds:

- **pairsim:** minimum threshold resemblance score for selecting known phosphorylation sites
- **th:** minimum threshold for creating a new segment, used in the step of "segmentation"
- **min_sim**: minimum similarity between matrix and the portion of the given protein sequence lps to be analysed, i. e. unknown functional protein sequence ups,
- **min_sup**: minimum support, denoting the number of known phosphorylation sites contained in one matrix
- **min_cons**: minimum conservation of matrices, used in "matrix construction"
- **mat_overlap**: minimum overlap between an existing matrix and a known phosphorylation site

Both variable input parameters and minimum threshold parameters may be user-defined or fixed.

Input data:

- Library of known phosphorylation sites (hierarchically structured)
- **lps:** longer protein sequence to be analysed

In the following an example for predicting phosphorylation sites according to the present invention is described in detail.

### Example 1

The protein sequence

$$\mathbf{u} = \text{LVVLTIISLIILIMLWQKKPRYEIRWKVIESVSSDGHEYIYVDPMQLPYD}$$

will be analysed in this example by aligning known phosphorylation sites. Each letter represents a type of amino acid. Therefore the following 19 proven phosphorylation sites, denominated by 051-A, A002-F, A002-C, A011-A, B110-B, A002-D, A065-B, A071-B, A002-I, A026-B, A039-A, A002-J, B281-C, B020-A, B012-A, B319-A, B054-A, B012-B, B046-B from the database, e.g. SWISS-PROT, are aligned to **u.** For each site the position in **u** is given by the variable **xp.** The variable **sim** *(resemblance)* shows the resemblance score calculated with Eq. 4.

```
seq:                    LVVLTIISLIILIMLWQKKPRYEIRWKVIESVSSDGHEYIYVDPMQLPYD
xp=567 xb=0 sim=12
1.3.0       +A051-A                             fpvsysssg
xp=573 xb=0 sim=18
1.3.0       +A002-F                                  sdggymdms
xp=574 xb=0 sim=162
1.3.0       +A002-C                                   dgheyiyvd
xp=574 xb=0 sim=32
1.3.0       +A011-A                                   kgheytnik
xp=574 xb=0 sim=18
1.3.0       +B110-B                                   kaEeyilkk
xp=576 xb=0 sim=162
1.3.0       +A002-D                                     heyiyvdPm
xp=576 xb=0 sim=12
1.3.0       +A065-B                                     nnyVyidPt
xp=576 xb=0 sim=12
1.3.0       +A071-B                                     nnyVyidPt
xp=584 xb=0 sim=18
1.3.0       +A002-I                                           ymapydnyv
xp=584 xb=0 sim=12
1.3.0       +A026-B                                          mmtpyvvtr
xp=587 xb=0 sim=18
1.3.0       +A002-J                                             pydnyvpsA
xp=556 xb=0 sim=18
1.1.0       +B281-C                    eslesyein
xp=560 xb=0 sim=20
1.1.0       +B020-A                      maevswkvl
xp=566 xb=0 sim=16
1.1.0       +B012-A                          eiveslsss
xp=566 xb=0 sim=12
1.1.0       +B054-A                         gvrqsrasd
xp=568 xb=0 sim=16
1.1.0       +B012-B                            veslssseE
xp=568 xb=0 sim=18
1.1.0       +B046-B                           lqryssdpt
```

Subsequently, all aligned proven phosphorylation sites are grouped by function which are denoted by 1.3.0 or 1.1.0 in the given example. Grouping by level 2 leads to two functional blocks: aligned proven phosphorylation sites of the groups 1.3.0 and 1.1.0. As the further processing for each functional block is the same only the further processing of block 1.3.0. is demonstrated.

The *aligned proven phosphorylation sites* (*apps*) of block 1.3.0. are grouped into positional segments in the next step. This is done by

(1) sorting all *apps* by their position **xp.**
(2) measuring the distance **d** between subsequently following *apps1* and *apps2*
(3) if **d** exceeds a certain threshold **th** than a new positional segment is created and **apps2** is its first member.

This shows the following example of the first two *apps* at positions **xp**=567 and **xp**=573:

```
xp=567 sim=12
1.3.0       A051-A                              fpvsysssg
xp=573 sim=18
1.3.0       A002-F                                   sdggymdms
```

Their positions (given by their **xp** coordinates) differ by 573-567= 8 letters. Assuming a user defined threshold **th** of 4, which for example represents half the desired matrix width, a first segment **G1** is created including the *apps* A051-A/xp=567 and a second segment **G2** is created including the *apps* A002-F/xp=573. Next the *apps* A002-F/xp=573 and A002-C/xp=574 are compared.

```
xp=573 sim=18
1.3.0      A002-F                              sdggymdms
xp=574  sim=162
1.3.0      A002-C                              dgheyiyvd
```

Their positions differ by 573-571= 2 letters. Assuming again a threshold **th** of 4 makes A002-C/xp=574 fall into the same segment **G2** of the *apps* A002-F/xp=573 now containing two *apps.* In the same way the following *apps* are compared.

```
xp=574 sim=162
1.3.0      A002-C                              dgheyiyvd
xp=574 sim=32
1.3.0      A011-A                              kgheytnik
```

Their positions xp differ by 0 enhancing **G2** (which is the respective segment for positions close to xp=573) to three *apps* now which are A002-F/xp=573, A002-X/xp=574, A011-A/xp=574. By this method the following *apps* are added to **G2:**

```
xp=574 sim=18
1.3.0      B110-B                              kaEeyilkk
xp=576 sim=162
1.3.0      A002-D                              heyiyvdPm
xp=576 sim=12
1.3.0      A065-B                              nnyVyidPt
xp=576 sim=12
1.3.0      A071-B                              nnyVyidPt
```

leading to the segment **G2** which finally contains seven *apps*: {A002-F/xp=573, A002-X/xp=574, A011-A/xp=574, B110-B/xp=574, A002-D/xp=576, A065-B/xp=576, A071-B/xp=576}.

As a next step the *apps* A071-B/xp=576 and A002-I/xp=584 will be compared:

```
xp=576 sim=12
1.3.0      A071-B                              nnyVyidPt
xp=584 sim=18
1.3.0      A002-I                              ymapydnyv
```

Their distance in positions is 8. As 8 exceeds the given threshold of 4 a new segment **G3** is created containing the *apps* A002-I/xp=584.

Next, the step of the matrix generation is described.

As an example matrices are constructed from segment **G2.** First, all *apps* from **G2** are sorted by their **sim** value which is their similarity to the protein sequence to be analysed yielding the following order of *apps*:

```
xp=574 sim=162
1.3.0      A002-C                              dgheyiyvd
xp=576 sim=162
1.3.0      A002-D                                heyiyvdPm
xp=574 sim=32
1.3.0      A011-A                              kgheytnik
xp=573 sim=18
1.3.0      A002-F                            sdggymdms
xp=574 sim=18
1.3.0      B110-B                               kaEeyilkk
xp=576 sim=12
1.3.0      A065-B                                 nnyVyidPt
xp=576 sim=12
1.3.0      A071-B                                 nnyVyidPt
```

From the first *apps* an initial matrix is build. Thus the initial matrix for G2 is constructed from:

```
xp=574 sim=162
1.3.0      A002-C                              dgheyiyvd
```

The matrix is constructed with a width specified by the parameter **matrixlen** which is user defined or given by the number of amino acids of the known phosphorylation sites, assumed in this example to be 9 amino acids. So the resulting initial matrix M1 has the following number of occurrences , denoted M1.fA, M1.fR, ..:

| Position | 1 | 2 | 3 | 4 | 5 | 6 | 7 | 8 | 9 |
|---|---|---|---|---|---|---|---|---|---|
| M1.fA | 0 | 0 | 0 | 0 | 0 | 0 | 0 | 0 | 0 |
| M1.fR | 0 | 0 | 0 | 0 | 0 | 0 | 0 | 0 | 0 |
| M1.fN | 0 | 0 | 0 | 0 | 0 | 0 | 0 | 0 | 0 |
| M1.fD | 1 | 0 | 0 | 0 | 0 | 0 | 0 | 0 | 1 |
| M1.fC | 0 | 0 | 0 | 0 | 0 | 0 | 0 | 0 | 0 |
| M1.fQ | 0 | 0 | 0 | 0 | 0 | 0 | 0 | 0 | 0 |
| M1.fE | 0 | 0 | 0 | 1 | 0 | 0 | 0 | 0 | 0 |
| M1.fG | 0 | 1 | 0 | 0 | 0 | 0 | 0 | 0 | 0 |
| M1.fH | 0 | 0 | 1 | 0 | 0 | 0 | 0 | 0 | 0 |
| M1.fI | 0 | 0 | 0 | 0 | 0 | 1 | 0 | 0 | 0 |
| M1.fL | 0 | 0 | 0 | 0 | 0 | 0 | 0 | 0 | 0 |
| M1.fK | 0 | 0 | 0 | 0 | 0 | 0 | 0 | 0 | 0 |
| M1.fM | 0 | 0 | 0 | 0 | 0 | 0 | 0 | 0 | 0 |
| M1.fF | 0 | 0 | 0 | 0 | 0 | 0 | 0 | 0 | 0 |
| M1.fP | 0 | 0 | 0 | 0 | 0 | 0 | 0 | 0 | 0 |
| M1.fS | 0 | 0 | 0 | 0 | 0 | 0 | 0 | 0 | 0 |
| M1.fT | 0 | 0 | 0 | 0 | 0 | 0 | 0 | 0 | 0 |
| M1.fW | 0 | 0 | 0 | 0 | 0 | 0 | 0 | 0 | 0 |
| M1.fY | 0 | 0 | 0 | 0 | 1 | 0 | 1 | 0 | 0 |

(continued)

| Position | 1 | 2 | 3 | 4 | 5 | 6 | 7 | 8 | 9 |
|----------|---|---|---|---|---|---|---|---|---|
| M1.fV | 0 | 0 | 0 | 0 | 0 | 0 | 0 | 1 | 0 |

**M1** has the leftmost position of its only *apps* A002-C/xp=574 which is 574. Following the next *apps* A002-D/xp=576

```
xp=576 sim=162
1.3.0       A002-D                                    heyiyvdPm
```

is examined for enhancing **M1.** The distance between the position xp=576 of A002-D/xp=576 and the.leftmost position of the matrix 574 is calculated to be 2. So A002-D/xp=576 overlaps the matrix by 6 letters. As the matrix has a width of 9 amino acids a complete overlap of 9 amino acids is required, which is not fulfilled here. Therefore A002-D/xp=576 is not used in **M1.** Instead another matrix **M2** is generated including A002-D/xp=576 and related to the respective portion of the unknown functional protein sequence **u**

```
1.3.0       A002-D                                    heyiyvdPm
```

leading to **M2**:

| Position | 1 | 2 | 3 | 4 | 5 | 6 | 7 | 8 | 9 |
|----------|---|---|---|---|---|---|---|---|---|
| M2.fA | 0 | 0 | 0 | 0 | 0 | 0 | 0 | 0 | 0 |
| M2.fR | 0 | 0 | 0 | 0 | 0 | 0 | 0 | 0 | 0 |
| M2.fN | 0 | 0 | 0 | 0 | 0 | 0 | 0 | 0 | 0 |
| M2.fD | 0 | 0 | 0 | 0 | 0 | 0 | 1 | 0 | 0 |
| M2.fC | 0 | 0 | 0 | 0 | 0 | 0 | 0 | 0 | 0 |
| M2.fQ | 0 | 0 | 0 | 0 | 0 | 0 | 0 | 0 | 0 |
| M2.fE | 0 | 1 | 0 | 0 | 0 | 0 | 0 | 0 | 0 |
| M2.fG | 0 | 0 | 0 | 0 | 0 | 0 | 0 | 0 | 0 |
| M2.fH | 1 | 0 | 0 | 0 | 0 | 0 | 0 | 0 | 0 |
| M2.fI | 0 | 0 | 0 | 1 | 0 | 0 | 0 | 0 | 0 |
| M2.fL | 0 | 0 | 0 | 0 | 0 | 0 | 0 | 0 | 0 |
| M2.fK | 0 | 0 | 0 | 0 | 0 | 0 | 0 | 0 | 0 |
| M2.fM | 0 | 0 | 0 | 0 | 0 | 0 | 0 | 0 | 1 |
| M2.fF | 0 | 0 | 0 | 0 | 0 | 0 | 0 | 0 | 0 |
| M2.fP | 0 | 0 | 0 | 0 | 0 | 0 | 0 | 1 | 0 |
| M2.fS | 0 | 0 | 0 | 0 | 0 | 0 | 0 | 0 | 0 |
| M2.fT | 0 | 0 | 0 | 0 | 0 | 0 | 0 | 0 | 0 |
| M2.fW | 0 | 0 | 0 | 0 | 0 | 0 | 0 | 0 | 0 |
| M2.fY | 0 | 0 | 1 | 0 | 1 | 0 | 0 | 0 | 0 |
| M2.fV | 0 | 0 | 0 | 0 | 0 | 1 | 0 | 1 | 0 |

**M2** has the leftmost position of its only *apps* A002-D/xp=576 which is 576. As the next of the apps of **G2** the apps A011-A/xp=574 is examined to merge with each of the existing matrices **M1** and **M2.**

```
xp=574 sim=32
1.3.0       A011-A                                          kgheytnik
```

As the apps A011-A/xp=574 completely overlaps the matrix **M1,** it is potentially merged to a temporary new matrix **M1'** now containing the two *apps:*

```
xp=574 sim=162
1.3.0       A002-C                                          dgheyiyvd
xp=574 sim=32
1.3.0       A011-A                                          kgheytnik
```

| Position | 1 | 2 | 3 | 4 | 5 | 6 | 7 | 8 | 9 |
|---|---|---|---|---|---|---|---|---|---|
| **M1'.fA** | 0 | 0 | 0 | 0 | 0 | 0 | 0 | 0 | 0 |
| **M1'.fR** | 0 | 0 | 0 | 0 | 0 | 0 | 0 | 0 | 0 |
| **M1'.fN** | 0 | 0 | 0 | 0 | 0 | 0 | 1 | 0 | 0 |
| **M1'.fD** | 1 | 0 | 0 | 0 | 0 | 0 | 0 | 0 | 1 |
| **M1'.fC** | 0 | 0 | 0 | 0 | 0 | 0 | 0 | 0 | 0 |
| **M1'.fQ** | 0 | 0 | 0 | 0 | 0 | 0 | 0 | 0 | 0 |
| **M1'.fE** | 0 | 0 | 0 | 2 | 0 | 0 | 0 | 0 | 0 |
| **M1'.fG** | 0 | 2 | 0 | 0 | 0 | 0 | 0 | 0 | 0 |
| **M1'.fH** | 0 | 0 | 2 | 0 | 0 | 0 | 0 | 0 | 0 |
| **M1'.fI** | 0 | 0 | 0 | 0 | 0 | 1 | 0 | 1 | 0 |
| **M1'.fL** | 0 | 0 | 0 | 0 | 0 | 0 | 0 | 0 | 0 |
| **M1'.fK** | 1 | 0 | 0 | 0 | 0 | 0 | 0 | 0 | 1 |
| **M1'.fM** | 0 | 0 | 0 | 0 | 0 | 0 | 0 | 0 | 0 |
| **M1'.fF** | 0 | 0 | 0 | 0 | 0 | 0 | 0 | 0 | 0 |
| **M1'.fP** | 0 | 0 | 0 | 0 | 0 | 0 | 0 | 0 | 0 |
| **M1'.fS** | 0 | 0 | 0 | 0 | 0 | 0 | 0 | 0 | 0 |
| **M1'.fT** | 0 | 0 | 0 | 0 | 0 | 1 | 0 | 0 | 0 |
| **M1'.fW** | 0 | 0 | 0 | 0 | 0 | 0 | 0 | 0 | 0 |
| **M1'.fY** | 0 | 0 | 0 | 0 | 2 | 0 | 1 | 0 | 0 |
| **M1'.fV** | 0 | 0 | 0 | 0 | 0 | 0 | 0 | 1 | 0 |

For **M1'** the average conservation is 87%. The conservation indicates the probability of the occurrence of a conserved amino acid at a specific position. The average conservation indicates the average probability of the occurrence of conserved amino acids in the examined sequence. The calculation of conservation is shown below.

For similarity and average conservation the thresholds **min_sim** = 1 and **min_cons** = 75%. are assumed. The threshold **min_sim** is evaluated if at least 3 sites are incorporated in the matrix, i. e. if the minimum support number min_sup of known phosphorylation sites apps in one matrix is achieved. If the average conservation value and the overall similarity exceeds both thresholds, **M1** is replaced by **M1'** [line 7 of Method 1 matrix_construction].
In the same manner, the *apps* (B110-B/xp=574)

```
xp=574 sim=18
1.3.0     B110-B                                          kaEeyilkk
```

is merged to **M1** resulting in the final matrix **M1** which includes the known phosphorylation sites apps

```
xp=574 sim=162
1.3.0     A002-C                                          dgheyiyvd
xp=574 sim=32
1.3.0     A011-A                                          kgheytnik
xp=574 sim=18
1.3.0     B110-B                                          kaEeyilkk
```

and leading to the matrix:

| Position | 1 | 2 | 3 | 4 | 5 | 6 | 7 | 8 | 9 |
|---|---|---|---|---|---|---|---|---|---|
| M1.fA | 0 | 1 | 0 | 0 | 0 | 0 | 0 | 0 | 0 |
| M1.fR | 0 | 0 | 0 | 0 | 0 | 0 | 0 | 0 | 0 |
| M1.fN | 0 | 0 | 0 | 0 | 0 | 0 | 1 | 0 | 0 |
| M1.fD | 1 | 0 | 0 | 0 | 0 | 0 | 0 | 0 | 1 |
| M1.fC | 0 | 0 | 0 | 0 | 0 | 0 | 0 | 0 | 0 |
| M1.fQ | 0 | 0 | 0 | 0 | 0 | 0 | 0 | 0 | 0 |
| M1.fE | 0 | 0 | 1 | 3 | 0 | 0 | 0 | 0 | 0 |
| M1.fG | 0 | 2 | 0 | 0 | 0 | 0 | 0 | 0 | 0 |
| M1.fH | 0 | 0 | 2 | 0 | 0 | 0 | 0 | 0 | 0 |
| M1.fI | 0 | 0 | 0 | 0 | 0 | 2 | 0 | 1 | 0 |
| M1.fL | 0 | 0 | 0 | 0 | 0 | 0 | 1 | 0 | 0 |
| M1.fK | 2 | 0 | 0 | 0 | 0 | 0 | 0 | 1 | 2 |
| M1.fM | 0 | 0 | 0 | 0 | 0 | 0 | 0 | 0 | 0 |
| M1.fF | 0 | 0 | 0 | 0 | 0 | 0 | 0 | 0 | 0 |
| M1.fP | 0 | 0 | 0 | 0 | 0 | 0 | 0 | 0 | 0 |
| M1.fS | 0 | 0 | 0 | 0 | 0 | 0 | 0 | 0 | 0 |
| M1.fT | 0 | 0 | 0 | 0 | 0 | 1 | 0 | 0 | 0 |
| M1.fW | 0 | 0 | 0 | 0 | 0 | 0 | 0 | 0 | 0 |
| M1.fY | 0 | 0 | 0 | 0 | 3 | 0 | 1 | 0 | 0 |
| M1.fV | 0 | 0 | 0 | 0 | 0 | 0 | 0 | 1 | 0 |

M1 has a *conservation* value of 80% as calculated as explained below. The overall similarity score of **M1** to the unknown protein sequence **u** is 25%, calculated by Eq. 4. In the same manner the second matrix **M2** is generated with

```
xp=576 sim=162
1.3.0      A002-D                                        heyiyvdPm
xp=576 sim=12
1.3.0      A065-B                                        nnyVyidPt
xp=576 sim=12
1.3.0      A071-B                                        nnyVyidPt
```

leading to

| Position | 1 | 2 | 3 | 4 | 5 | 6 | 7 | 8 | 9 |
|---|---|---|---|---|---|---|---|---|---|
| M2.fA | 0 | 0 | 0 | 0 | 0 | 0 | 0 | 0 | 0 |
| M2.fR | 0 | 0 | 0 | 0 | 0 | 0 | 0 | 0 | 0 |
| M2.fN | 2 | 2 | 0 | 0 | 0 | 0 | 0 | 0 | 0 |
| M2.fD | 0 | 0 | 0 | 0 | 0 | 0 | 3 | 0 | 0 |
| M2.fC | 0 | 0 | 0 | 0 | 0 | 0 | 0 | 0 | 0 |
| M2.fQ | 0 | 0 | 0 | 0 | 0 | 0 | 0 | 0 | 0 |
| M2.fE | 0 | 1 | 0 | 0 | 0 | 0 | 0 | 0 | 0 |
| M2.fG | 0 | 0 | 0 | 0 | 0 | 0 | 0 | 0 | 0 |
| M2.fH | 1 | 0 | 0 | 0 | 0 | 0 | 0 | 0 | 0 |
| M2.fI | 0 | 0 | 0 | 1 | 0 | 2 | 0 | 0 | 0 |
| M2.fL | 0 | 0 | 0 | 0 | 0 | 0 | 0 | 0 | 0 |
| M2.fK | 0 | 0 | 0 | 0 | 0 | 0 | 0 | 0 | 0 |
| M2.fM | 0 | 0 | 0 | 0 | 0 | 0 | 0 | 0 | 1 |
| M2.fF | 0 | 0 | 0 | 0 | 0 | 0 | 0 | 0 | 0 |
| M2.fP | 0 | 0 | 0 | 0 | 0 | 0 | 0 | 3 | 0 |
| M2.fS | 0 | 0 | 0 | 0 | 0 | 0 | 0 | 0 | 0 |
| M2.fT | 0 | 0 | 0 | 0 | 0 | 0 | 0 | 0 | 2 |
| M2.fW | 0 | 0 | 0 | 0 | 0 | 0 | 0 | 0 | 0 |
| M2.fY | 0 | 0 | 3 | 0 | 3 | 0 | 0 | 0 | 0 |
| M2.fV | 0 | 0 | 0 | 2 | 0 | 1 | 0 | 0 | 0 |

with an *overall_similarity* of 3.125% and a *conservation* of 88%.

A third matrix **M3** which is not shown here is constructed only from

```
xp=573 sim=18
1.3.0 ¨    A002-F                                        sdggymdms
```

The support of this matrix **M3** is 1 and does not exceed the given minimum support threshold of 3 and is therefore too low so that the matrix M3 is discarded in a support checking step following the matrix generation. The matrices **M1** and **M2** include a higher number of assigned known phosphorylation sites apps and are retained because their support exceeds the given minimum support threshold.

Finally, the following results are achieved:

Protein sequence to be analysed:

```
                      LVVLTIISLIILIMLWQKKPRYEIRWKVIESVSSDGHEYIYVDPMQLPYD

Phase 4
1.3.0        574  582                                      DnnEYnnnD
1.3.0        A002-C                                        DGHEYIYVD
1.3.0        A011-A                                        KGHEYTNIK
1.3.0        B110-B                                        KAEEYILKK
; Sim=25.0002; Conservation=80

                                                          |||||||||
                                                          |||||||||
                                                          |||||||||
                                                          |||||||||
                                                          |||||||||


XX
1.3.0        576  584                                      NNYnYnDPn
1.3.0        A002-D                                        HEYIYVDPM
1.3.0        A065-B                                        NNYVYIDPT
1.3.0        A071-B                                        NNYVYIDPT
; Sim=3.12508; Conservation=88

                                                          |||||||||
                                                          |||||||||
                                                          |||||||||
                                                          |||||||||
                                                          |||||||||


XX
Segments:
1.3.0        574  584                                      ===phospho=
```

As used herein "phospho" denotes a phosphorylation group or group of phosphorylation sites which consists of all phosphorylation sites which are not comprised in an annotated or named group (such as contained in a databank). Examples for such annotated groups are PKC and PKA.

The calculation of the conservation value for a given matrix is illustrated for the following example matrix:

| Position | 1 | 2 | 3 | 4 | 5 | 6 | 7 | 8 | 9 |
|---|---|---|---|---|---|---|---|---|---|
| M2.fA | 0 | 0 | 0 | 0 | 0 | 0 | 0 | 0 | 0 |
| M2.fR | 0 | 0 | 0 | 0 | 0 | 0 | 0 | 0 | 0 |
| M2.fN | 2 | 2 | 0 | 0 | 0 | 0 | 0 | 0 | 0 |
| M2.fD | 0 | 0 | 0 | 0 | 0 | 0 | 3 | 0 | 0 |
| M2.fC | 0 | 0 | 0 | 0 | 0 | 0 | 0 | 0 | 0 |
| M2.fQ | 0 | 0 | 0 | 0 | 0 | 0 | 0 | 0 | 0 |
| M2.fE | 0 | 1 | 0 | 0 | 0 | 0 | 0 | 0 | 0 |
| M2.fG | 0 | 0 | 0 | 0 | 0 | 0 | 0 | 0 | 0 |
| M2.fH | 1 | 0 | 0 | 0 | 0 | 0 | 0 | 0 | 0 |
| M2.fI | 0 | 0 | 0 | 1 | 0 | 2 | 0 | 0 | 0 |
| M2.fL | 0 | 0 | 0 | 0 | 0 | 0 | 0 | 0 | 0 |
| M2.fK | 0 | 0 | 0 | 0 | 0 | 0 | 0 | 0 | 0 |
| M2.fM | 0 | 0 | 0 | 0 | 0 | 0 | 0 | 0 | 1 |
| M2.fF | 0 | 0 | 0 | 0 | 0 | 0 | 0 | 0 | 0 |
| M2.fP | 0 | 0 | 0 | 0 | 0 | 0 | 0 | 3 | 0 |

(continued)

| Position | 1 | 2 | 3 | 4 | 5 | 6 | 7 | 8 | 9 |
|---|---|---|---|---|---|---|---|---|---|
| M2.fS | 0 | 0 | 0 | 0 | 0 | 0 | 0 | 0 | 0 |
| M2.fT | 0 | 0 | 0 | 0 | 0 | 0 | 0 | 0 | 2 |
| M2.fW | 0 | 0 | 0 | 0 | 0 | 0 | 0 | 0 | 0 |
| M2.fY | 0 | 0 | 3 | 0 | 3 | 0 | 0 | 0 | 0 |
| M2.fV | 0 | 0 | 0 | 2 | 0 | 1 | 0 | 0 | 0 |

For each matrix value the probability is calculated leading to the matrix:

| Position | 1 | 2 | 3 | 4 | 5 | 6 | 7 | 8 | 9 |
|---|---|---|---|---|---|---|---|---|---|
| M2.fA | 0 | 0 | 0 | 0 | 0 | 0 | 0 | 0 | 0 |
| M2.fR | 0 | 0 | 0 | 0 | 0 | 0 | 0 | 0 | 0 |
| M2.fN | 0,67 | 0,67 | 0 | 0 | 0 | 0 | 0 | 0 | 0 |
| M2.fD | 0 | 0 | 0 | 0 | 0 | 0 | 1 | 0 | 0 |
| M2.fC | 0 | 0 | 0 | 0 | 0 | 0 | 0 | 0 | 0 |
| M2.fQ | 0 | 0 | 0 | 0 | 0 | 0 | 0 | 0 | 0 |
| M2.fE | 0 | 0,33 | 0 | 0 | 0 | 0 | 0 | 0 | 0 |
| M2.fG | 0 | 0 | 0 | 0 | 0 | 0 | 0 | 0 | 0 |
| M2.fH | 0,33 | 0 | 0 | 0 | 0 | 0 | 0 | 0 | 0 |
| M2.fI | 0 | 0 | 0 | 0,33 | 0 | 0,67 | 0 | 0 | 0 |
| M2.fL | 0 | 0 | 0 | 0 | 0 | 0 | 0 | 0 | 0 |
| M2.fK | 0 | 0 | 0 | 0 | 0 | 0 | 0 | 0 | 0 |
| M2.fM | 0 | 0 | 0 | 0 | 0 | 0 | 0 | 0 | 0,33 |
| M2.fF | 0 | 0 | 0 | 0 | 0 | 0 | 0 | 0 | 0 |
| M2.fP | 0 | 0 | 0 | 0 | 0 | 0 | 0 | 1 | 0 |
| M2.fS | 0 | 0 | 0 | 0 | 0 | 0 | 0 | 0 | 0 |
| M2.fT | 0 | 0 | 0 | 0 | 0 | 0 | 0 | 0 | 0,67 |
| M2.fW | 0 | 0 | 0 | 0 | 0 | 0 | 0 | 0 | 0 |
| M2.fY | 0 | 0 | 1 | 0 | 1 | 0 | 0 | 0 | 0 |
| M2.fV | 0 | 0 | 0 | 0,67 | 0 | 0,33 | 0 | 0 | 0 |

Now the weighted information content of each matrix value $I_{IA}$ is calculated using the formula

$$I_{IA} = p_{IA} * log_{10} p_{IA}.$$

Here $log_{10}$ instead of $log_2$ is used which makes no differences in evaluating the matrix.

| Position | 1 | 2 | 3 | 4 | 5 | 6 | 7 | 8 | 9 |
|---|---|---|---|---|---|---|---|---|---|
| M2.fA | -0 | -0 | -0 | -0 | -0 | -0 | -0 | -0 | -0 |
| M2.fR | -0 | -0 | -0 | -0 | -0 | -0 | -0 | -0 | -0 |
| M2.fN | -0,12 | -0,12 | -0 | -0 | -0 | -0 | -0 | -0 | -0 |

(continued)

| Position | 1 | 2 | 3 | 4 | 5 | 6 | 7 | 8 | 9 |
|---|---|---|---|---|---|---|---|---|---|
| M2.fD | -0 | -0 | -0 | -0 | -0 | -0 | 0 | -0 | -0 |
| M2.fC | -0 | -0 | -0 | -0 | -0 | -0 | -0 | -0 | -0 |
| M2.fQ | -0 | -0 | -0 | -0 | -0 | -0 | -0 | -0 | -0 |
| M2.fE | -0 | -0,16 | -0 | -0 | -0 | -0 | -0 | -0 | -0 |
| M2.fG | -0 | -0 | -0 | -0 | -0 | -0 | -0 | -0 | -0 |
| M2.fH | -0,16 | -0 | -0 | -0 | -0 | -0 | -0 | -0 | -0 |
| M2.fI | -0 | -0 | -0 | -0,16 | -0 | -0,12 | -0 | -0 | -0 |
| M2.fL | -0 | -0 | -0 | -0 | -0 | -0 | -0 | -0 | -0 |
| M2.fK | -0 | -0 | -0 | -0 | -0 | -0 | -0 | -0 | -0 |
| M2.fM | -0 | -0 | -0 | -0 | -0 | -0 | -0 | -0 | -0,16 |
| M2.fF | -0 | -0 | -0 | -0 | -0 | -0 | -0 | -0 | -0 |
| M2.fP | -0 | -0 | -0 | -0 | -0 | -0 | -0 | 0 | -0 |
| M2.fS | -0 | -0 | -0 | -0 | -0 | -0 | -0 | -0 | -0 |
| M2.fT | -0 | -0 | -0 | -0 | -0 | -0 | -0 | -0 | -0,12 |
| M2.fW | -0 | -0 | -0 | -0 | -0 | -0 | -0 | -0 | -0 |
| M2.fY | -0 | -0 | 0 | -0 | 0 | -0 | -0 | -0 | -0 |
| M2.fV | -0 | -0 | -0 | -0,12 | -0 | -0,16 | -0 | -0 | -0 |
| 87,98442575 | 78,546 | 78,546 | 99,782 | 78,546 | 99,782 | 78,546 | 99,782 | 99,782 | 78,546 |

For each column the to 100% normalized sum of all

$$C_l = \log_{10}(20) + \sum_{lA} p_{lA} * \log_{10}(p_{lA})$$

Is given. The average conservation value is calculated by

$$conservation := 1/20 * \sum_l C_l = 87,98442575 \%$$

As described in Eq. 4 the overall similarity is calculated with:

$$\textbf{overallsim} := overall\_similarity(ups, m) := \prod_{l=1}^{s} \left(\frac{p_{lAl}}{p_{lO}}\right).$$

and evaluated for the following matrix and *ups* = DGHEYIYVD.

| Position | 1 | 2 | 3 | 4 | 5 | 6 | 7 | 8 | 9 |
|---|---|---|---|---|---|---|---|---|---|
| M1.fA | 0 | 1 | 0 | 0 | 0 | 0 | 0 | 0 | 0 |
| M1.fR | 0 | 0 | 0 | 0 | 0 | 0 | 0 | 0 | 0 |
| M1.fN | 0 | 0 | 0 | 0 | 0 | 0 | 1 | 0 | 0 |

(continued)

| Position | 1 | 2 | 3 | 4 | 5 | 6 | 7 | 8 | 9 |
|---|---|---|---|---|---|---|---|---|---|
| M1.fD | 1 | 0 | 0 | 0 | 0 | 0 | 0 | 0 | 1 |
| M1.fC | 0 | 0 | 0 | 0 | 0 | 0 | 0 | 0 | 0 |
| M1.fQ | 0 | 0 | 0 | 0 | 0 | 0 | 0 | 0 | 0 |
| M1.fE | 0 | 0 | 1 | 3 | 0 | 0 | 0 | 0 | 0 |
| M1.fG | 0 | 2 | 0 | 0 | 0 | 0 | 0 | 0 | 0 |
| M1.fH | 0 | 0 | 2 | 0 | 0 | 0 | 0 | 0 | 0 |
| M1.fI | 0 | 0 | 0 | 0 | 0 | 2 | 0 | 1 | 0 |
| M1.fL | 0 | 0 | 0 | 0 | 0 | 0 | 1 | 0 | 0 |
| M1.fK | 2 | 0 | 0 | 0 | 0 | 0 | 0 | 1 | 2 |
| M1.fM | 0 | 0 | 0 | 0 | 0 | 0 | 0 | 0 | 0 |
| M1.fF | 0 | 0 | 0 | 0 | 0 | 0 | 0 | 0 | 0 |
| M1.fP | 0 | 0 | 0 | 0 | 0 | 0 | 0 | 0 | 0 |
| M1.fS | 0 | 0 | 0 | 0 | 0 | 0 | 0 | 0 | 0 |
| M1.fT | 0 | 0 | 0 | 0 | 0 | 1 | 0 | 0 | 0 |
| M1.fW | 0 | 0 | 0 | 0 | 0 | 0 | 0 | 0 | 0 |
| M1.fY | 0 | 0 | 0 | 0 | 3 | 0 | 1 | 0 | 0 |
| M1.fV | 0 | 0 | 0 | 0 | 0 | 0 | 0 | 1 | 0 |

The value $p_{IAI}$ equals the number of occurrence of the amino acid of the respective position in the protein sequence to be analysed.

The value $p_{IO}$ is the maximum number of occurrence of one of the amino acids of the respective column, this leads to the following table

| Position | 1 | 2 | 3 | 4 | 5 | 6 | 7 | 8 | 9 |
|---|---|---|---|---|---|---|---|---|---|
| $p_{IAI}$ | 1 | 2 | 2 | 3 | 3 | 2 | 1 | 1 | 1 |
| $p_{IO}$ | 2 | 2 | 2 | 3 | 3 | 2 | 1 | 1 | 2 |

and therefore the similarity is:

$$\text{sim} = \frac{72}{288} = 0.25.$$

In the following another example is illustrated.

## Example 2

The database of proven phosphorylation sites (pps) is hierarchically structured in three levels. The first level defines the kind of post-translational-modification (here 1 = phosphorylation sites). The second level refers to the phosphorylated residue (1=S, 2=T, 3=Y). The third level defines the acting kinase (1.1.2 = Phosphorylation, at S, PKA is acting). A zero is used to indicate, if the kinase is not known (1.1.0 = phosphorylation at S of an unknown kinase). In the following excerpts of the total database are shown. Each known phosphorylation site has its name extended, describing its the database origin (>B034-A16@P11217_15 indicates that B034-A16 stems from SWISS-PROT database, protein P11217, aminoacid 15).

PKA
1.1.2.

```
>B034-A16@P11217_15
KQGSGRGL
>B034-A02@P11217_15
KRKQISVRGL
>B034-A17@P11217_15
KRKQGSVRGL
>B034-A04@P11217_15
KQISVRGL
>B034-A18@P11217_15
KRKQISGRGL
>B034-A07@P11217_15
RKQISVR
>B034-A19@P11217_15
RKEISVR
>B034-A21@P11217_15
RKQITVR
>B034-A10@P11217_15
KAKQISVRGL
>B034-A11@P11217_15
KKQISVR
>B034-A12@P11217_15
KRAQISVRGL
>B034-A14@P11217_15
KRKQISVAGL
>B034-A15@P11217_15
KRKQISVGGL


EGFR (autophosphorylation)
1.3.5.
>B046-I@P06268_1172
DNPDYQQDF
>B046-J@P06268_1197
ENAEYLRVA
>B046-F@P06268_1016
DADEYLIPQ
>B046-G@P06268_1092
PVPEYINQS
>B046-H@P06268_1110
QNPVYHNQP


phospho
1.1.0.
>B060-A@P14598_303
PPRRSSIRN
>B060-B@P14598_304
PRRSSIRNA
>B060-C@P14598_315
IHQRSRKRL
>B060-E@P14598_328
YRRNSVRFL
>B060-F@P14598_345
PGPQSPGSP
>B060-G@P14598_348
QSPGSPLEE
>B060-H@P14598_379
LNRCSESTK
```

The following output represents an example with more proven phosphorylation sites segmented by both categories: class (acceptor residues) and subclass (kinase). Kinases belonging to the subclasses PKC, PKA and p34cdc2 and also unknown kinases phospho are used in the matrix construction.

```
=======================================================================
seq(   0..   59)       GSSKSKPKDPSQRRRSLEPPDSTHHGGFPASQTPNKTAAPDTHRTPSRSFGTVATEPKLF
-----------------------------------------------------------------------
Phase 3
1.1.0      42    50                                                      nnnPSnnnn
1.1.0      +A001-C                                                       HRTPSRSFG
1.1.0      +B046-A                                                       PLTPSGEAP
1.1.0      +B094-G                                                       ERSPSPSFR
1.1.0      +B146-A                                                       LLRPSRRVR
; Sim=50.0003; Conservation =78
                                                                         |||||||||
                                                                         |||||||||
                                                                         |||||||||
                                                                         |||||||||
                                                                          ||||||||

XX
1.1.1       6    14             nnnnSnRRn
1.1.1      +A001-A              PKDPSQRRR
1.1.1      +B007-A              QKRPSQRSK
1.1.1      +B030-B              PRRVSRRRR
1.1.1      +B116-B              TQSTSGRRR
1.1.1      +B117-C              PRRVSRRRR
1.1.1      +B176-A              METPSQRRA
1.1.1      +B177-A              METPSQRRA
; Sim=33.3336; Conservation =78
                               |||||||||
                               |||||||||
                               |||||||||
                               |||||||||
                               |||||||||      .
XX
1.1.2      11    19                 nRRnSnnnn
1.1.2      +A001-B                   QRRRSLEPP
1.1.2      +B021-A                   RRRGSSIPQ
1.1.2      +B028-A                   AVRRSDRAY
1.1.2      +B049-B                   GRRQSLIQD
1.1.2      +B064-B                   SRKRSGEAT
1.1.2      +B066-B                   TTRRSCSKT
1.1.2      +B067-B                   KSRPSLPLP
1.1.2      +B091-B                   LCRRSTTDC
1.1.2      +B092-A                   LRRFSLATM
1.1.2      +B093-A                   LRRFSLATM
1.1.2      +B100-A                   PRRDSTEGF
1.1.2      +B101-A                   MRRNSFTPL
1.1.2      +B103-A                   AARLSLTDP
1.1.2      +B110-A                   PRRRSSFGI
1.1.2      +B114-D                   ERRKSHEAE
1.1.2      +B115-B                   SRRDSLFVP
1.1.2      +B135-B                   ERTNSLPPV
1.1.2      +B135-C                   QRRTSLTGS
1.1.2      +B135-E                   SRRSSLGSL
1.1.2      +B145-C                   PRMPSLSVP
1.1.2      +B233-A                   QRRHSLEPP
; Sim=100; Conservation=75
                                    |||||||||
                                    |||||||||
                                    |||||||||
                                    |||||||||
                                     ||  |
XX
```

```
1.2.3       28   36                              nnnnTPNKn
1.2.3       +A001-E                              PASQTPNKT
1.2.3       +B267-D                              GGTGTPNKE
1.2.3       +B268-F                              SASGTPNKE
; Sim=25.0003; Conservation =86

                                                 ||||||||||
                                                 ||||||||||
                                                 ||||||||||
                                                 ||||||||||
                                                 ||||||||||

XX
Segments:
1.1.1       6    14         ====PKC==
1.1.2       11   19            ====PKA==
1.2.3       28   36                              ==p34cdc2
1.1.0       42   50                                             ==phospho
```

The features of the present invention disclosed in the specification, the claims and/or the drawings may both separately and in any combination thereof be material for realizing the invention in various forms thereof.

**Claims**

1.  Method for the detection of an unknown functional protein sequence within a given protein sequence having a number of amino acids comprising the following steps:

    a) providing said given protein sequence having a number of amino acids wherein functional sites shall be predicted;

    b) providing a number of known functional protein sequences having a number of contiguous amino acids wherein said known functional protein sequences include proven functional sites;

    c) for each of said known functional protein sequences, evaluating a resemblance score at one ore more alignment positions between the known functional protein sequences and a portion of said given protein sequence which is to be analysed;

    d) if said resemblance scores exceeds a minimum resemblance threshold, selecting the known functional protein sequence(s) and their alignment position(s);

    e) assigning each of the selected known functional protein sequence to a segment of said given protein sequence according to their alignment position(s) wherein said segment is defined by said one or more alignment positions;

    f) for each of the segments, creating a matrix containing the number of occurrences of each amino acid at a specific position of said given protein sequence aligned with the respective segment,

    g) for each of the matrices, evaluating an overall similarity between the known functional protein sequences assigned to the respective segment and the portion of the given protein sequence related to said evaluated segment, and evaluating a conservation value rating the occurrence of conserved amino acids within the evaluated segment.

2.  Method according to claim 1, wherein the alignment positions of one of said known functional protein sequences are selected if the resemblance score of one of said known functional protein sequence has a maximum resemblance score relating to said maximum resemblance scores at all alignment positions.

3.  Method according to claim 1 or 2 wherein the step c) of evaluating said resemblance scores at one ore more alignment positions includes the evaluation of said resemblance scores at all alignment positions.

4.  Method according to any of claims 1 to 3, wherein according to step e) said selected known functional protein sequences are assigned into the same segment if their selected alignment positions are equal.

5.  Method according to any of claims 1 to 3, wherein according to step e) said selected known functional protein sequences are assigned into the same segment if their selected alignment positions are within a predetermined segmentation range.

**6.** Method according to claim 5, wherein successive known functional protein sequences are assigned to the same segment if the distance of said successive known functional protein sequences is less than half of the matrix length.

**7.** Method according to any of claims 1 to 6, wherein the evaluation of the overall similarity of step g) is performed only if the number of known functional protein sequences assigned to one segment exceeds a predetermined threshold number of known functional protein sequences, otherwise the segment is discarded.

**8.** Method according to claim 7, wherein the predetermined threshold number of known functional protein sequences is at least 3.

**9.** Method according to any of claims 1 to 8, wherein the evaluation of the overall similarity of step g) further includes the step of

for each of said matrices, evaluating said overall similarity using the formula

$$\textbf{overallsim} := overall\_similarity(ups, \text{m}) := \prod_{l=1}^{s} \; (\frac{p_{lAl}}{p_{lO}}).$$

wherein ups is the specific portion of the given protein sequence aligned with the respective segment,
wherein m is the matrix,
wherein s is the length of the specific portion of the given protein sequence,
wherein p10 is the maximum number of occurrences of a specific amino acid, and
wherein $p_{lAl}$ is the number of occurrences of the amino acid at the respective position of the specific portion
of the given protein sequence.

**10.** Method according to any of claims 1 to 9, wherein said evaluating of said conservation value *conservation* rating the occurrence of conserved amino acids within the evaluated segment is performed using the formula

$$conservation := 1/s * \sum_{l} \; (\log_{base} (20) + \sum_{lA} \; p_{lA} * \log_{base}(p_{lA}))$$

wherein s is the total number of possible amino acids for each of the positions,
wherein base is the basis of the logarithm, preferably 2 or 10,
wherein $p_{lA}$ denotes the evaluated probability of the occurrences of the specific amino acid in the respective column 1 of said matrix.

**11.** Method according to one of the claims 1 to 10 wherein the step e) of assigning each of the selected known functional protein sequence to a segment of said given protein sequence according to their alignment position(s) is performed if said overall similarity of the segment including the assigned known functional protein sequence exceeds the overall similarity of the segment without said known functional protein sequence, and/or if the conservation value of said segment including the assigned known functional protein sequence exceeds the conservation value of said segment without said assigned known functional protein sequence.

**12.** Method for verification of an unknown functional protein sequence comprising the steps of the method according to any of claims 1 to 11 resulting in an annotated unknown functional protein sequence whereby the annotated unknown functional protein sequence is a functional protein sequence having a minimum overall similarity and/or a minimum conservation value,
further comprising the step of

experimentally verifying the annotated unknown functional protein sequence.

**13.** Use of the method according to any of claims 1 to 12 for predicting phosphorylation sites in a given protein sequence, wherein the known functional protein sequences contain proven phosphorylation sites.

**14.** Apparatus for the detection of an unknown functional protein sequence within a given protein sequence, wherein said given protein sequence includes a number of amino acids wherein functional sites shall be predicted, wherein a number of known functional protein sequences having proven functional sites are provided which include a number of contiguous amino acids, comprising:

- means for evaluating a resemblance score between the known functional protein sequences and a portion to be analysed of said given protein sequence at one ore more alignment positions for each of said known functional protein sequences;
- selecting means to select the known functional protein sequence and their alignment positions, if said resemblance scores exceed a minimum resemblance threshold,
- assigning means to assign each of the selected known functional protein sequences to a segment according to their alignment position wherein said segment is defined by said one or more alignment positions,
- overall similarity evaluating means to evaluate the overall similarity between the known functional protein sequences assigned to the respective segment and the portion of the given protein sequence related to said evaluated segment for each of the segments.

**15.** Apparatus according to claim 14, further comprising:

- overall conservation evaluating means to evaluate the conservation value for each of the segments wherein the conservation value rates the occurrence of conserved amino acids within the evaluated segment.

Fig. 1

lps

ups 1 (1.1.1)
& ups 2 (1.1.2)

ups 3 (1.1.1)
& ups 4 (1.1.2)

block of apps of
function 1.1.1.

block of apps of
function 1.1.2.

1 apps

segment with apps of one
function in one block

EP 1 339 006 A1

pps

selection & alignment

apps

functional grouping

blocks

positional grouping

segments

matrix construction

matrices

Fig. 2

**European Patent Office**

# EUROPEAN SEARCH REPORT

**Application Number**

EP 02 00 3469

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | GRABE NIELS: "AliBaba2: Context specific identification of transcription factor binding sites." IN SILICO BIOLOGY, 'Online! vol. 1, 2000, XP002196811 Online Journal Retrieved from the Internet: <URL:http://www.bioinfo.de/isb/2000/01/0019/> 'retrieved on 2002-04-10! * the whole document * | 1-15 | G06F19/00 |
| A | BUCHER ET AL: "A flexible motif search technique based on generalized profiles" COMPUTERS AND CHEMISTRY, PERGAMON PRESS, OXFORD, GB, vol. 20, no. 1, 1996, pages 3-23, XP002107535 ISSN: 0097-8485 * the whole document * | 1-15 | |
| A | BLOM NIKOLAJ ET AL: "Sequence and structure-based prediction of eukaryotic protein phosphorylation sites." JOURNAL OF MOLECULAR BIOLOGY, vol. 294, no. 5, 17 December 1999 (1999-12-17), pages 1351-1362, XP001068460 ISSN: 0022-2836 * the whole document * | 1-15 | **TECHNICAL FIELDS SEARCHED (Int.Cl.7)**<br><br>G06F |
| A | SAQI MONSOOR A S ET AL: "PdbMotif-A tool for the automatic identification and display of motifs in protein structures." COMPUTER APPLICATIONS IN THE BIOSCIENCES, vol. 10, no. 5, 1994, pages 545-546, XP002196813 ISSN: 0266-7061 * the whole document * | 1-15 | |

-/--

The present search report has been drawn up for all claims

| Place of search | Date of completion of the search | Examiner |
|---|---|---|
| MUNICH | 16 May 2002 | Wimmer, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

**Application Number**

EP 02 00 3469

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | MARCOTTA E M ET AL: "A COMBINED ALGORITHM FOR GENOMEWIDE PREDICTION OF PROTEIN FUNCTION" NATURE, MACMILLAN JOURNALS LTD. LONDON, GB, vol. 402, no. 6757, 4 November 1999 (1999-11-04), pages 83-86, XP002935757 ISSN: 0028-0836 * the whole document * | 1-15 | |
| A | EP 1 013 759 A (JAPAN SCIENCE & TECH CORP) 28 June 2000 (2000-06-28) * the whole document * | 1-15 | |

TECHNICAL FIELDS SEARCHED (Int.Cl.7)

The present search report has been drawn up for all claims

| Place of search | Date of completion of the search | Examiner |
|---|---|---|
| MUNICH | 16 May 2002 | Wimmer, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**                    EP 02 00 3469

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2002

| Patent document cited in search report | Publication date | Patent family member(s) | Publication date |
|---|---|---|---|
| EP 1013759 A | 28-06-2000 | JP 10210979 A | 11-08-1998 |
| | | JP 10222486 A | 21-08-1998 |
| | | JP 11155578 A | 15-06-1999 |
| | | JP 11213003 A | 06-08-1999 |
| | | EP 1013759 A1 | 28-06-2000 |
| | | WO 9833900 A1 | 06-08-1998 |

EPO FORM P0459

For more details about this annex : see Official Journal of the European Patent Office, No. 12/82